Europäisches Patentamt

European Patent Office

Office européen des brevets

(11) Veröffentlichungsnummer: **0 072 401**

A1

(12) **EUROPÄISCHE PATENTANMELDUNG**

(21) Anmeldenummer: 82105360.0

(22) Anmeldetag: 18.06.82

(51) Int. Cl.³: **C 07 C 120/08**

(30) Priorität: 13.08.81 DE 3131968

(43) Veröffentlichungstag der Anmeldung:
23.02.83 Patentblatt 83/8

(84) Benannte Vertragsstaaten:
DE GB NL

(71) Anmelder: CHEMISCHE WERKE HÜLS AG
- RSP Patente / PB 15 - Postfach 13 20
D-4370 Marl 1(DE)

(72) Erfinder: Hofmann, Peter, Dr.
Lipper Weg 193
D-4370 Marl(DE)

(54) Verfahren zur Herstellung von aliphatischen Nitrilen.

(57) Verfahren zur Herstellung von aliphatischen Nitrilen durch Umsetzung von entsprechenden Carbonsäuren oder Carbonsäurealkylestern mit Ammoniak in Gegenwart eines metallhaltigen Katalysators, wobei man die Umsetzung bei einer Temperatur von 150 bis 290°C in Gegenwart von Eisen oder einer Eisenverbindung als Katalysator durchführt.

EP 0 072 401 A1

Croydon Printing Company Ltd.

Verfahren zur Herstellung von aliphatischen Nitrilen

Aliphatische Nitrile werden in industriellem Maßstab fast ausschließlich durch Umsetzung von Fettsäuren bzw. Derivaten dieser Fettsäuren mit Ammoniak gewonnen. Bei einer Arbeitsweise in der Gasphase geht man dabei so vor, daß man die Dämpfe der Fettsäure bzw. ihrer Derivate, insbesondere Ester, zusammen mit Ammoniak bei Temperaturen zwischen 320 und 600 °C über dehydratisierend wirkende Kontakte leitet. Als solche kommen in Frage Aluminiumoxid, Silicagel, Oxide des Thoriums, Titans, Molybdäns, Wolframs und Vanadins. (US-PSS 1 991 955, 2 177 619, 2 205 076; J. A. Mitchell, E. E. Reid, J. Amer. Chem. Soc. 53, 321 (1931); J. Appl. Chem. USSR, 45, 1824 (1972).

Die hohe thermische Belastung der Einsatzstoffe und der Reaktionsprodukte sowie des zur Durchführung der Reaktion benötigten Apparates können reduziert werden, wenn man in flüssiger Phase arbeitet. Allerdings erfordert auch die Durchführung der Reaktion in flüssiger Phase noch Temperaturen bis zu 350 °C, die bei Verwendung niedriger siedender Ausgangsstoffe eine Durchführung der Reaktion bei erhöhtem Druck nötig machen (US-PSS 2 061 314, 2 546 521, 2 555 606; R. L. Kenyon, D. V. Stingley, H. P. Young, Ind. Eng. Chem. 42, 202 (1950).

Eine weitere Erniedrigung der Reaktionstemperatur bei Arbeiten in flüssiger Phase wird ermöglicht durch die Anwendung spezieller Katalysatoren. In Gegenwart von Alkoholaten des Titans, Zirkons oder Hafniums sind Reaktionstemperaturen von nicht über 315 °C erforderlich (US-PS 2 993 926), bei Verwendung von Kobaltsalzen als Katalysator kann die maximale Reaktionstemperatur auf 290 °C begrenzt werden (US-PS 2 493 637). Die beiden letztgenannten Verfahren sind jedoch durch den Nachteil belastet, daß sie die Benutzung relativ teurer und nur in begrenztem Maße wiederverwendbarer Katalysatoren erfordern.

Aufgabe der vorliegenden Erfindung war es daher, ein Verfahren zur Herstellung von aliphatischen Nitrilen durch Umsetzung entsprechender Carbonsäuren oder Carbonsäurealkylester zu entwickeln, das bei einer Arbeitsweise in flüssiger Phase und Einhaltung von Temperaturen, die im allgemeinen unter denen der Verfahren des zitierten Standes der Technik liegen, die Verwendung preisgünstiger, die Wirtschaftlichkeit des Verfahrens nicht beeinträchtigender Katalysatoren gestattet.

Diese Aufgabe wurde überraschenderweise durch die in den Patentansprüchen beschriebene Maßnahme gelöst.

Überraschend deswegen, weil es gemäß den Ausführungen in der US-PS 2 205 076 auf Seite 3, linke Spalte, Zeilen 17 bis 19, nicht zu erwarten war, daß Eisen und Eisenverbindungen eine positive katalytische Wirkung ausüben. Nach den Ausführungen in der US-PS 2 205 076 begünstigt Eisen nämlich unerwünschte Nebenreaktionen, wie z. B. das Cracken der Produkte. Aus diesem Grunde wird vorgeschlagen, Apparate aus Aluminium und vorzugsweise Nickel zu verwenden.

Überraschend ist ferner, daß der erfindungsgemäße Katalysator die Umsetzung von Fettsäuren und Fettsäureestern mit nahezu gleichen Ergebnissen gestattet. Die wenigen literaturbekannten Beispiele, die sich mit einer Nitrilherstellung auf Basis von Fettsäureestern befassen und hierzu Daten mitteilen, zeigen, daß der Einsatz von Estern stets mit gravierenden Nachteilen verbunden ist: Von J. A. Mitchell und E. E. Reid (J. Amer. Chem. Soc. 53, 321 (1931) wird berichtet, daß die Lebensdauer des für die Nitrilherstellung in Gasphase verwendeten Katalysators, beim Einsatz von Estern im Vergleich zum Einsatz freier Säuren stark abnimmt. Im Gegensatz zu dem auf Basis von Fettsäuren normalerweise hohe Ausbeuten an Nitril liefernden Flüssigphasenverfahren, werden bei Verwendung von Methylestern als Ausgangsprodukt nur 60 bis 65 % Ausbeute erzielt (P. B. Jonardhan, J. Sci. Ind. Research (India), 9B, 208 (1950).

Die Umsetzung von Fettsäureestern in Gegenwart von Alkoholaten des Titans, Zirkons oder Hafniums gemäß dem Verfahren der US-PS 2 993 926 kann, insbesondere bei Vorliegen von Estern niederer Alkohole, wie z. B. Methanol oder Ethanol, infolge Umesterung mit dem Katalysator zur Bildung flüchtiger Alkoholate des Titans, Zirkons oder Hafniums führen, die leicht mit Ammoniak aus dem Reaktionsgemisch ausgetragen werden können.

Auch diese Verfahrensmaßnahme ist also zumindest beim Einsatz von Fettsäureestern keineswegs uneingeschränkt anwendbar.

Geeignete Carbonsäuren für das erfindungsgemäße Verfahren sind einmal und bevorzugt geradkettige und verzweigte gesättigte aliphatische Monocarbonsäuren mit 4 bis 22 Kohlenstoffatomen, zum anderen aber auch ungesättigte aliphatische Monocarbonsäuren und aliphatische Dicarbonsäuren innerhalb dieses C-Zahl-Bereiches. Weiterhin können die Alkylester der genannten Carbonsäuren mit bis zu 8, vorzugsweise bis zu 4 Kohlenstoffatomen im Alkanolrest eingesetzt werden.

Typische Vertreter sind z. B. Butter-, Valerian-, Capron-, Önant-, Capryl-, Pelargon-, Caprin-, Laurin-, Myristin-, Palmitin-, Stearin-, Arachin-, Behen-, Öl-, Kork- und Dodecandisäure sowie die Ester dieser Säuren mit Methanol, Ethanol, n-Propanol, iso-Propanol, n-Butanol, iso-Butanol und Pentanol.

Die für das erfindungsgemäße Verfahren geeigneten Carbonsäuren und Carbonsäurealkylester können z. B. durch Verseifung bzw. Umesterung nativer Rohstoffe (Öle und Fette)
hergestellt werden. Ebenso geeignet sind jedoch Carbonsäuren bzw. Carbonsäurealkylester, die auf Basis petrochemischer Syntheseverfahren gewonnen werden. Als solche
kommen z. B. in Frage die Hydrocarboxylierung oder Hydrocarboxyalkylierung von Olefinen (DE-AS 29 12 489), die
Hydroformylierung von Olefinen mit anschließender Oxidation der primären Reaktionsprodukte zu Carbonsäuren (J.
Falbe, New Syntheses with Carbon Monoxide, Springer-Verlag, Berlin, Heidelberg, New York, 1980) und die Oxidation von Paraffinen (H. Weissermel, H.-J. Arpe, Industrielle Organische Chemie, 2. Auflage, Seite 196, Verlag Chemie, Weinheim, New York, 1978).

Als für das erfindungsgemäße Verfahren geeignete Katalysatoren haben sich Verbindungen des Eisens sowie Eisen
selbst erwiesen. Geeignete Eisenverbindungen sind z. B.
Salze des Eisens mit anorganischen oder organischen Säuren, wie z. B. Chloride, Nitrate, Sulfate, Acetate,
Naphthenate, Laurate, Palmitate, Stearate oder Oxide des
Eisens, wie z. B. $Fe_2O_3$. Es spielt dabei keine Rolle, in
welcher Wertigkeitsstufe das Eisen in den Eisenverbindungen vorliegt, d. h. es können sowohl Verbindungen des 2-
als auch des 3wertigen Eisens eingesetzt werden. Beim
Einsatz von Eisen ist es vorteilhaft, das Metall in möglichst feinverteilter Form, z. B. in Form von Spänen oder
Pulvern einzusetzen.

Die Menge des als Eisenverbindung bzw. als Eisenmetall
eingesetzten Katalysators beträgt im allgemeinen 0,01 bis
5, vorzugsweise 0,3 bis 3 Gewichtsprozent Eisen, bezogen
auf die Carbonsäure bzw. den Carbonsäurealkylester.

Die Umsetzung der Carbonsäuren bzw. der Carbonsäurealkylester mit Ammoniak in Gegenwart des Katalysators wird bei dem erfindungsgemäßen Verfahren bei einer Temperatur von 150 bis 290 °C, vorzugsweise von 180 bis 265 °C, durchgeführt. Das bedeutet, daß das erfindungsgemäße Verfahren beim Einsatz solcher Carbonsäuren und Carbonsäurealkylester im Prinzip drucklos durchgeführt werden kann, deren Siedepunkt bei Normaldruck > 150 °C beträgt. Eine besonders einfache Verfahrensweise, die einen Verzicht auf druckfeste Apparaturen gestattet, ergibt sich stets dann, wenn der Siedepunkt des jeweiligen Substrates (Carbonsäure oder Carbonsäurealkylester) bei Normaldruck oberhalb von 150 °C liegt und die Reaktionstemperatur den Siedepunkt nicht überschreitet.

Eine gebräuchliche Ausführungsform des erfindungsgemäßen Verfahrens besteht darin, daß man das Substrat zusammen mit dem darin gelösten oder suspendierten Katalysator in einem Rührkessel vorlegt und in der Hitze solange Ammoniak einleitet bis eine vollständige Umsetzung erfolgt ist. Das nicht verbrauchte Ammoniak kann nach Kondensation des mitausgetragenen Wassers bzw. Wasser/Alkanol-Gemisches wieder in die Reaktion zurückgeführt werden. Neben dieser diskontinuierlichen Fahrweise sind selbstverständlich auch Ausführungsformen möglich, die eine kontinuierliche Durchführung des Verfahrens gestatten.

Die nach dem erfindungsgemäßen Verfahren hergestellten Nitrile könne z. B. als Lösungsmittel, Weichmacher in Synthesefasern und Textilhilfsmittel verwendet werden. Die aus den Nitrilen durch Hydrierung leicht erhältlichen Amine finden z. B. als Ausgangsprodukte zur Herstellung von kationischen Tensiden, Emulgatoren und Korrosionsinhibitoren Verwendung.

Das erfindungsgemäße Verfahren wird durch die nachfolgenden Beispiele näher erläutert.

Beispiele 1 bis 8

Für die Nitrilherstellung wurde ein Gemisch von isomeren Dodecansäuremethylestern (53 Mol-%) mit einem n-Anteil von 77 Gew.-% und Tridecansäuremethylestern (47 Mol-%) mit einem n-Anteil von 75 Gew.-% verwendet, das durch Hydrocarboxylierung eines Gemisches isomerer n-Undecene und n-Dodecene ($\alpha$-Anteil jeweils $<$ 1 Gew.-%) gemäß dem Verfahren der DE-AS 29 12 489 hergestellt wurde.

Die Hydrocarboxylierungsreaktion wurde unter Einhaltung folgender Mengenverhältnisse

    0,5 Mol n-Undecene
    0,5 Mol n-Dodecene
    2    Mole Methanol
    0,4 Mol $\gamma$-Picolin
    0,04 Grammatom Cobalt in Form eines 10 Gew.-%
    Cobalt enthaltenden Cobaltnaphthenats

in einem 50 l Autoklaven unter den folgenden Reaktionsbedingungen durchgeführt:

    Reaktionstemperatur:        185 $^{\circ}$C
    Kohlenmonoxiddruck:         180 bar
    (Co enthält 1,5 Vol.-% $H_2$)
    Reaktionsdauer:             55 min.

Aus dem unter diesen Bedingungen erhaltenen Reaktionsgemisch wurde das in den Beispielen 1 bis 8 eingesetzte Estergemisch durch Destillation gewonnen. Es wurde mit den in Tabelle 1 aufgeführten Katalysatoren in einer solchen Menge versetzt, daß 1 Gew.-% Metall, bezogen auf das Estergemisch, anwesend war. Bei einer Reaktionstemperatur von 250 $^{\circ}$C wurde pro Stunde 1/10 der stöchiometrischen Menge $NH_3$ eingeleitet. Der Reaktionsfortschritt wurde ersichtlich aus der Menge des Alkohol/Wassergemisches, das zusammen mit nicht umgesetztem $NH_3$ ausgetragen und anschließend kondensiert wurde und konnte außerdem durch die gaschromatographische Analyse des Reaktionsgemisches verfolgt werden.

Tabelle 1

| Beispiel-Nr. | 1 | 2 | 3 | 4 |
|---|---|---|---|---|
| Katalysator | Fe-Pulver | Fe-III-nitrat | Fe-Oxid ($Fe_2O_3$) | Fe-III-chlorid |
| Reakt.Zeit für vollständ. Ums. (h) | 15 | 14 | 14 | 13 |
| Beispiel-Nr. | 5 | 6 | 7 | 8 |
| Katalysator | Fe-II-sulfat | Fe-III-sulfat | Fe-laurat[1] | Fe-naphthenat[1] |
| Reakt.Zeit für vollständ. Ums. (h) | 13 | 13 | 12 | 12 |

[1] Fe-Gehalt: 10 Gew.-%

Beispiele 9 bis 16

Unter den Bedingungen von Beispiel 8 wurden die in Tabelle 2 aufgeführten Substrate zu Nitrilen umgesetzt.

Tabelle 2

| Beispiel-Nr. | 9 | 10 | 11 | 12 | 13 | 14 | 15 | 16 |
|---|---|---|---|---|---|---|---|---|
| Substrat | Laurin-säure-methyl-ester | Stearin-säure-methyl-ester | Laurin-säure-ethyl-ester | Laurin-säure-butyl-ester | Pelar-gon-säure | Gemisch aus 50 Mol-% Dodecansäure (n-Ant. 77 Gew.-%) u. 50 Mol-% Tridecansäure (n-Ant. 75 Gew.-%) | Öl-säure | Dodecan-disäure |
| Reakt.Zeit für voll-ständ. Ums. (h) | 12 | 14 | 12 | 14 | 12 | 12 | 14 | 13 |

## Beispiele 17 bis 19

Beispiel 9 wurde bei unterschiedlichen Reaktionstemperaturen wiederholt.

## Tabelle 3

| Beispiel-Nr. | 17 | 18 | 19 |
|---|---|---|---|
| Reaktionstemperatur ($^o$C) | 200 | 230 | 260 |
| Reakt.Zeit für vollständ. Ums. (h) | 20 | 15 | 12 |

## Beispiele 20 und 21

Beispiel 9 wurde für unterschiedliche Katalysatorkonzentrationen (reines Metall in Gew.-%, bezogen auf Substrat) wiederholt.

## Tabelle 4

| Beispiel-Nr. | 20 | 21 |
|---|---|---|
| Katalysatorkonz. (Gew.-%) | 0,5 | 2 |
| Reakt.Zeit für vollständ. Ums. (h) | 16 | 14 |

## Beispiel 22

Beispiel 9 wurde wiederholt mit der Ausnahme, daß pro Stunde 1/6 der stöchiometrischen Menge $NH_3$ eingeleitet wurde. Die zur vollständigen Umsetzung benötigte Zeit betrug 7,5 Stunden.

<u>Beispiel 23</u>

Das nach Beispiel 8 erhaltene Reaktionsgemisch wird destillativ aufgearbeitet. Der dabei erhaltene Rückstand, der das gesamte als Katalysator eingesetzte Eisen enthielt, wurde erneut unter den Bedingungen des Beispiels 8 als Katalysator verwendet. Die für eine vollständige Umsetzung erforderliche Reaktionszeit betrug wieder 12 Stunden.

Die nach den Beispielen 1 bis 23 nach vollständiger Umsetzung erhaltenen Nitrile hatten eine Reinheit $\geq$ 95 Gew.-%.

Patentansprüche:

1. Verfahren zur Herstellung von aliphatischen Nitrilen durch Umsetzung von entsprechenden Carbonsäuren oder Carbonsäurealkylestern mit Ammoniak in Gegenwart eines metallhaltigen Katalysators, dadurch gekennzeichnet, daß man die Umsetzung bei einer Temperatur von 150 bis 290 °C in Gegenwart von Eisen oder einer Eisenverbindung als Katalysator durchführt.

2. Verfahren nach Anspruch 1, dadurch gekennzeichnet, daß man die Umsetzung bei einer Temperatur von 180 bis 265 °C durchführt.

## EINSCHLÄGIGE DOKUMENTE

| Kategorie | Kennzeichnung des Dokuments mit Angabe soweit erforderlich. der maßgeblichen Teile | Betrifft Anspruch | KLASSIFIKATION DER ANMELDUNG (Int. Cl. ³) |
|---|---|---|---|
| X,Y | EP-A-0 000 916 (HOECHST) *Seite 20, Beispiel 9; Seiten 1-3* | 1,2 | C 07 C 120/08 |
| X,Y | US-A-2 229 219 (H.F.OXLEY) *Spalten 1,2* | 1,2 | |
| X,Y | US-A-2 439 426 (W.F.GRESHAM) *Spalte 3* | 1,2 | |
| Y | FR-A- 986 870 (SINNOUA) *Seite 1; Seite 3* | 1 | |

RECHERCHIERTE SACHGEBIETE (Int. Cl. ³)

C 07 C 120/00

Der vorliegende Recherchenbericht wurde für alle Patentansprüche erstellt.

| Recherchenort | Abschlußdatum der Recherche | Prüfer |
|---|---|---|
| DEN HAAG | 21-10-1982 | VERHULST W. |